# FASCICULE DE BREVET EUROPEEN

(11) **EP 1 898 820 B1**
(45) Date de publication et mention de la délivrance du brevet: **21.10.2015**
(21) Numéro de dépôt: 06778742.4
(22) Date de dépôt: 30.06.2006
(51) Int. Cl.: A61B 17/88, A61B 17/02

(54) **INSTRUMENT POUR LE DEPLACEMENT D'UNE VERTEBRE**
INSTRUMENT ZUR BEWEGUNG EINES WIRBELS
INSTRUMENT FOR MOVING A VERTEBRA

(30) Priorité: 01.07.2005 FR 0552004
(43) Date de publication de la demande: 19.03.2008
(73) Titulaire: Spinevision S.A., 75012 Paris (FR)
(72) Inventeur: FREZAL, Olivier, F-93110 Rosny sous Bois (FR); PETIT, Dominique, F-62180 Verton (FR); BETTE, Stéphane, F-75011 Paris (FR)
(74) Mandataire: Fidal Innovation
(86) Numéro de dépôt international: PCT/FR2006/001554
(87) Numéro de publication internationale: WO 2007/003785

(56) Documents cités:
- EP-A- 0 499 037
- EP-A- 0 602 351
- WO-A-95/05786
- US-A- 5 782 831

## Description

L'invention concerne les instruments pour la correction de déformation de la colonne vertébrale et concerne plus particulièrement un instrument pour le déplacement d'une vertèbre par rapport à une autre.

L'invention est particulièrement destinée au traitement de spondylolisthésis. Le spondylolisthésis est un déplacement vers l'avant d'une vertèbre par rapport à celle du dessous. Cette pathologie possède des conséquences neurologiques importantes provoquant douleur et perte de la mobilité des membres inférieurs dans les cas les plus graves. Le vieillissement ou certaines maladies congénitales peuvent provoquer cette pathologie.

Des techniques permettant de traiter cette pathologie sont connues. Il existe notamment une technique appelée arthrodèse consistant à fixer en place ou à replacer la vertèbre déplacée à l'aide de vis pédiculaires et d'éléments de liaisons longitudinaux puis à fusionner cette vertèbre avec la vertèbre adjacente. La fusion postéro latérale de la vertèbre avec la vertèbre adjacente est réalisée par avivement de l'os et apport de greffon. Cette arthrodèse postéro latérale peut éventuellement être complétée par une fusion inter somatique avec insertion d'un greffon et éventuellement d'un implant ou cage intersomatique entre les corps vertébraux.

Afin de corriger les spondylolisthesis, il est connu dans l'art antérieur des instruments et des implants permettant le déplacement des vertèbres. Cependant, ces instruments ou ces implants imposent une seule cinématique de déplacement de la vertèbre. Ainsi, ils ne s'adaptent pas facilement à l'anatomie particulière du patient. Particulièrement, lors du déplacement de la vertèbre, l'effort de traction nécessaire peut être très important du fait de la cinématique imposée par l'instrument ou l'implant qui ne correspond pas toujours au trajet de moindre effort. Notamment, la vertèbre que l'on souhaite déplacer peut avoir besoin de basculer dans un premier temps pour franchir l'obstacle constitué par le plateau vertébral de la vertèbre adjacente, avant d'être ramenée en arrière dans un deuxième temps Par conséquent, il existe des risques d'échec lors de cette manoeuvre, notamment le risque d'arracher les vis pédiculaires ou de briser les pédicules. Ces risques sont d'autant plus importants que le patient est âgé et que sa qualité osseuse est faible. En outre, les instruments et implants de l'art antérieur ne permettent pas de libérer un accès vers l'espace discal pour réaliser une fusion inter somatique.

La demande de brevet européen EP 0 499 037 tente de limiter ces risques en proposant un dispositif de réalignement des vertèbres comprenant deux bras aptes à être articulés. Lesdits bras peuvent ainsi, préalablement à la manoeuvre de correction, être positionnés sur les vertèbres correspondantes en tenant compte de l'anatomie du patient et de la position de la vertèbre déplacée. Cependant, une fois le préréglage effectué, les bras sont bloqués dans la position choisie et la vertèbre déplacée sous contrainte.

On connaît également du brevet américain US 5 782 831 un instrument destiné à réduire le déplacement d'une vertèbre disposée entre des vertèbres adjacentes. Ledit dispositif de réduction comprend un bras destiné à être fixé sur un élément de liaison longitudinal monté sur des vis pédiculaires préalablement fixées à des vertèbres disposées de part et d'autre de la vertèbre déplacée. Un tel instrument présente cependant un certain nombre d'inconvénients. En particulier, sa mise en oeuvre requiert la présence de deux vertèbres au moins, disposées de part et d'autre de la vertèbre déplacée, afin de permettre la fixation de l'élément de liaison longitudinal. Un tel instrument ne permet donc pas le réalignement d'une vertèbre d'extrémité.

L'invention vise à remédier à ces problèmes en proposant un instrument pour le déplacement d'une vertèbre par rapport à une autre par une cinématique non contrainte, en s'adaptant à l'anatomie particulière de chaque patient et en laissant l'accès libre vers l'espace discal pour réaliser une fusion intersomatique.

A cet effet, et selon un premier aspect, l'invention propose un instrument pour le déplacement d'une vertèbre par rapport à une autre comprenant:
- une potence composée d'une traverse sensiblement horizontale et d'un premier bras dont l'extrémité inférieure possède des moyens d'ancrage à une première vertèbre; et
- un second bras dont l'extrémité inférieure possède des moyens d'ancrage à une seconde vertèbre, et une extrémité supérieure possède un moyen d'actionnement en translation dudit second bras selon une direction sensiblement perpendiculaire à la traverse, ledit moyen d'actionnement étant monté sur la traverse,
ledit instrument étant caractérisé en ce que le second bras est déformable selon au moins un degré de liberté entre l'extrémité inférieure du second bras et son moyen d'actionnement.

Ainsi, une force antéro-postérieure est exercée entre les deux vertèbres tout en conservant une liberté de mouvement à la seconde vertèbre par rapport à la première. Par conséquent, les contraintes sur les vertèbres sont diminuées, et le désalignement entre l'extrémité inférieure et le moyen d'actionnement du second bras est compensé.

Par ailleurs, l'instrument, tel que configuré, ne nécessite le « sacrifice » que d'une seule vertèbre pour réaligner la vertèbre déplacée. Par le terme « sacrifice », on entend l'utilisation d'une vertèbre en place pour l'ancrage de l'instrument, le redressement de la vertèbre déplacée s'effectuant alors par rapport à la vertèbre portant l'instrument.

En outre, l'instrument offre également un certain nombre d'ajustements de positions qui lui permettent de s'adapter à l'anatomie du patient et laissent au chirurgien l'accès au disque intervertébral pour la réalisation de la fusion inter somatique. Par exemple, il est possible d'ajuster angulairement et longitudinalement la position du moyen d'actionnement du second bras par rapport à l'extrémité inférieure du premier bras. Il est également possible de décaler latéralement la traverse par rapport à l'extrémité inférieure du premier bras.

En outre, il est également possible d'effectuer la manoeuvre de correction et de maintenir la correction obtenue sans que l'élément de liaison longitudinale destiné à la fixation et à la fusion osseuse ne soit en place. Cette possibilité est particulièrement intéressante car elle laisse au chirurgien l'accès au disque intervertébral pour la réalisation de l'arthrodèse intersomatique, et ce à tout moment de la manoeuvre de correction.

Après la correction et l'éventuelle arthrodèse intersomatique, le chirurgien peut connecter l'élément de liaison longitudinale aux vis pédiculaires et même appliquer des manoeuvres de compression/distraction lorsque l'instrument est en place, la correction antéro-postérieure étant conservée. Une fois que la position finale de vertèbres est jugée satisfaisante, le chirurgien peut la fixer par serrage des éléments de liaison longitudinale sur les vis pédiculaires, et ensuite seulement peut retirer l'instrument pour le déplacement d'une vertèbre.

D'autres objets et avantages de l'invention apparaîtront au cours de la description qui suit, faite en référence aux dessins annexés, dans lesquels :
- la figure 1 représente un schéma de principe de l'instrument selon l'invention;
- la figure 2 représente une vue explosée de l'instrument selon l'invention ;
- les figures 3 et 4 représentent des vues explosées en perspective de l'instrument ;

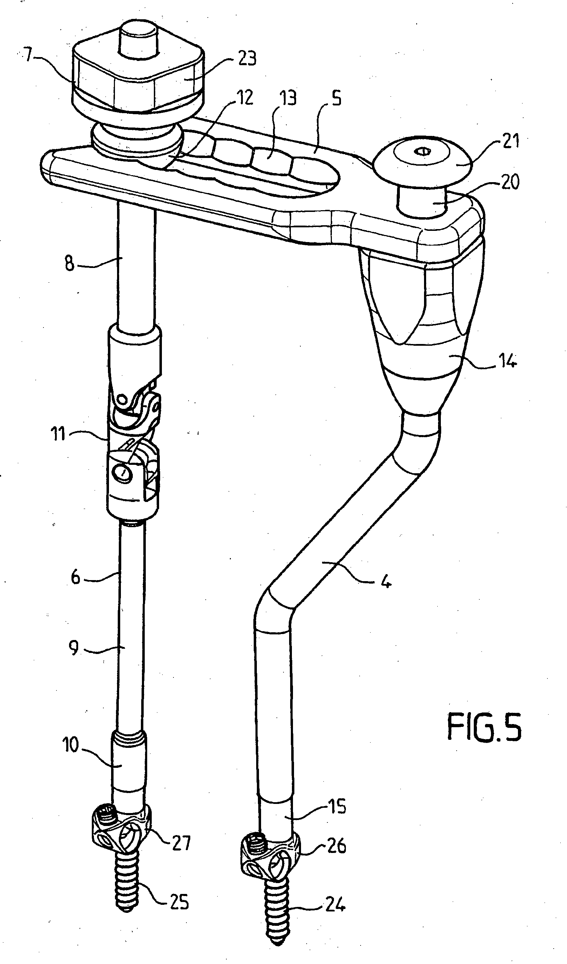

- la figure 5 représente une vue en perspective de l'instrument selon l'invention ;
- la figure 6 représente une vue en perspective de l'instrument, les connecteurs accueillant un élément de liaison longitudinal ; et
- la figure 7 représente une vue détaillée des connecteurs accueillant un élément de liaison longitudinale.

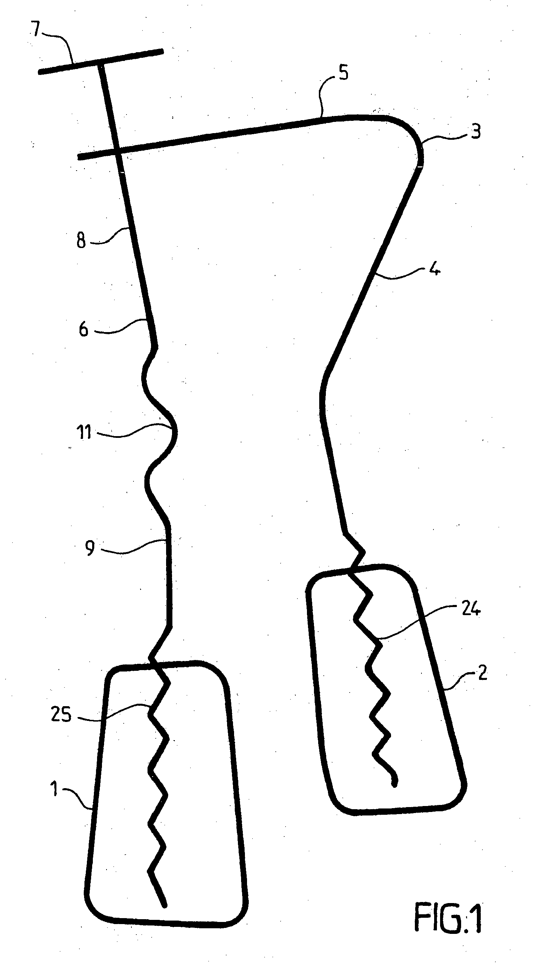

L'instrument pour le déplacement d'une vertèbre 1 par rapport à une autre vertèbre 2, tel qu'il est représenté sur la figure 1, est composé d'une potence 3. Cette potence 3 est composée d'un premier bras 4 vertical et d'une traverse horizontale 5. Dans le mode de réalisation représentée, la potence 3 possède une poignée 14 permettant une bonne prise en main de l'instrument.

L'extrémité inférieure 15 du premier bras 4 possède des moyens d'ancrage 24 à une première vertèbre 2.

L'instrument comprend également un second bras 6 dont une extrémité inférieure 10 possède des moyens d'ancrage 25 à une seconde vertèbre 1, et une extrémité supérieure possède un moyen d'actionnement 7 en translation dudit second bras 6 selon une direction sensiblement perpendiculaire à la traverse 5. Le moyen d'actionnement 7 est monté sur la traverse 5.

Les moyens d'ancrage 24, 25 des bras 4, 6 sur les vertèbres 1, 2 sont des vis pédiculaires.

Dans un mode de réalisation, les vis pédiculaires sont positionnées dans des logements disposés aux extrémités inférieures 10,15 des bras.

Dans un autre mode de réalisation de l'invention, les vis pédiculaires sont positionnées dans des logements de connecteurs 26, 27 solidarisables aux extrémités inférieures 10, 15 des bras. A cet effet, les connecteurs possèdent des filetages permettant de les solidariser aux extrémités inférieures 10, 15 des bras.

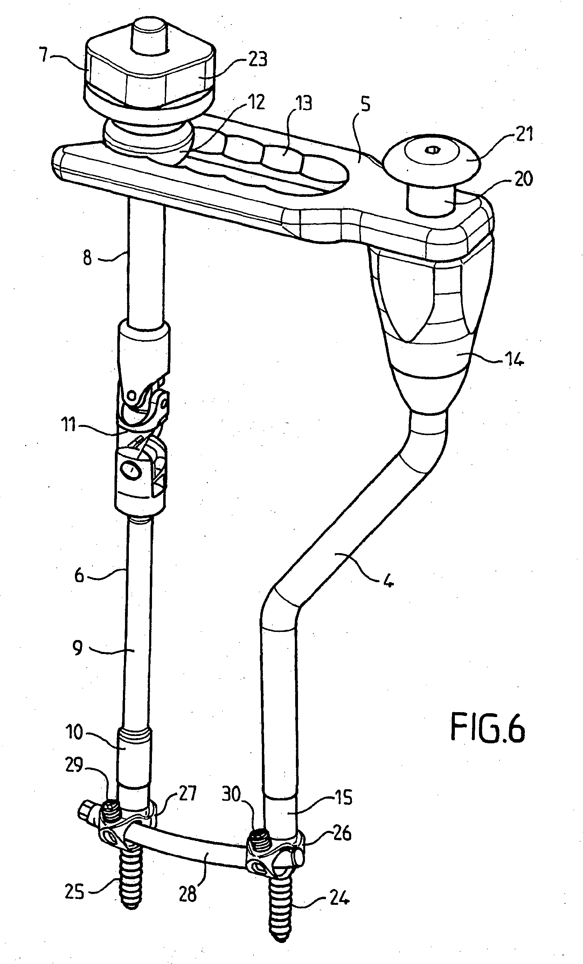

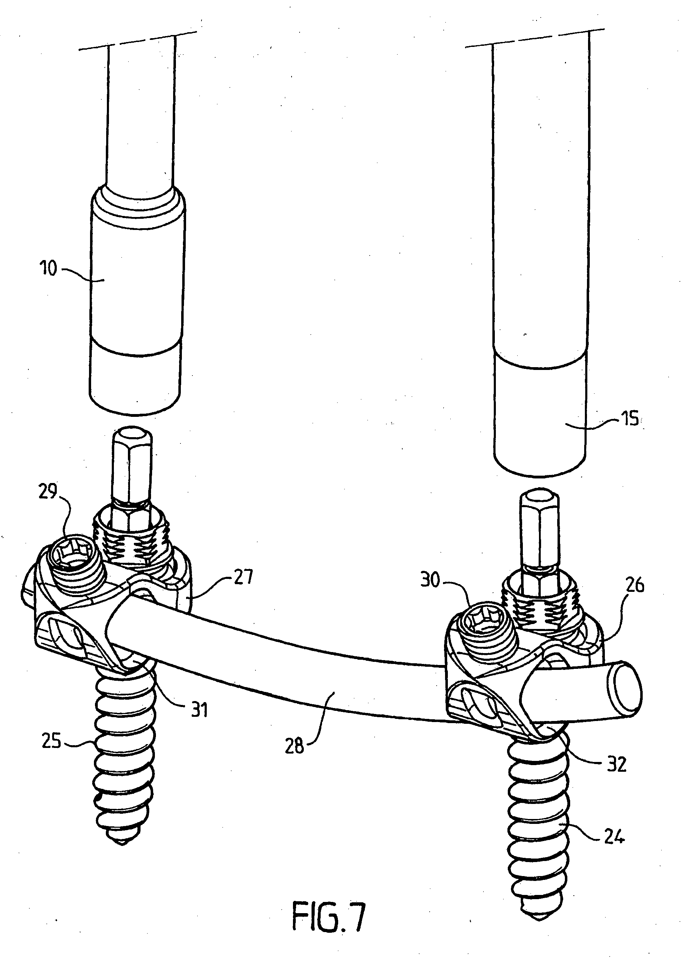

Notons que, avantageusement, les vis pédiculaires sont également utilisées pour la fixation d'un élément de liaison longitudinal 28, illustrées sur les figures 6 et 7, permettant la fixation des vertèbres entre elles et la fusion osseuse. L'élément de liaison 28 est mis en place après la manoeuvre de correction. Avantageusement, tel qu'il est représenté sur les figures 6 et 7, les connecteurs 26, 27 possèdent des logements 31, 32 permettant de recevoir l'élément de liaison longitudinale 28 et des vis 29, 30 permettent de bloquer l'élément de liaison 28 dans les logements 31, 32.

Le second bras 6 est déformable selon au moins un degré de liberté. Le degré de liberté permet de ne pas imposer une cinématique de déplacement à la seconde vertèbre 1. Ainsi, le déplacement de la seconde vertèbre 1 s'adapte à l'anatomie de la colonne vertébrale du patient car elle suit une trajectoire de moindre effort lors de la translation du second bras 6.

En outre, le désalignement entre l'extrémité inférieure 10 du second bras 6 et son moyen d'actionnement 7 est compensé. Par conséquent, le moyen d'actionnement 7 peut être disposé en désalignement avec l'extrémité 10 du second bras sans provoquer de contraintes mécaniques sur la seconde vertèbre 1 et sur les vis. La position du moyen d'actionnement 7 peut alors être ajusté.

Dans un mode de réalisation de l'invention non représenté, au moins une partie du second bras 6 est réalisé dans un matériau souple permettant sa déformation. Cette partie souple peut être un câble, une pièce métallique déformable ou être réalisée dans un matériau polymère.

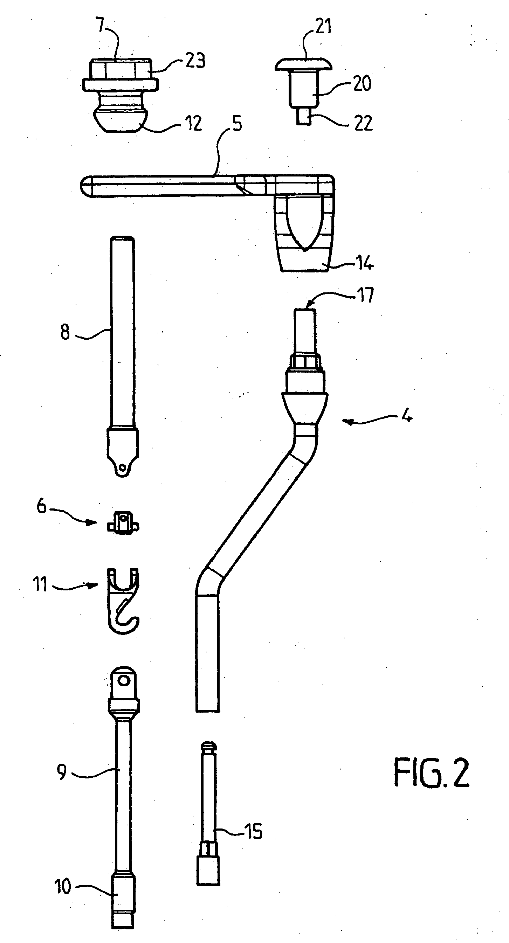

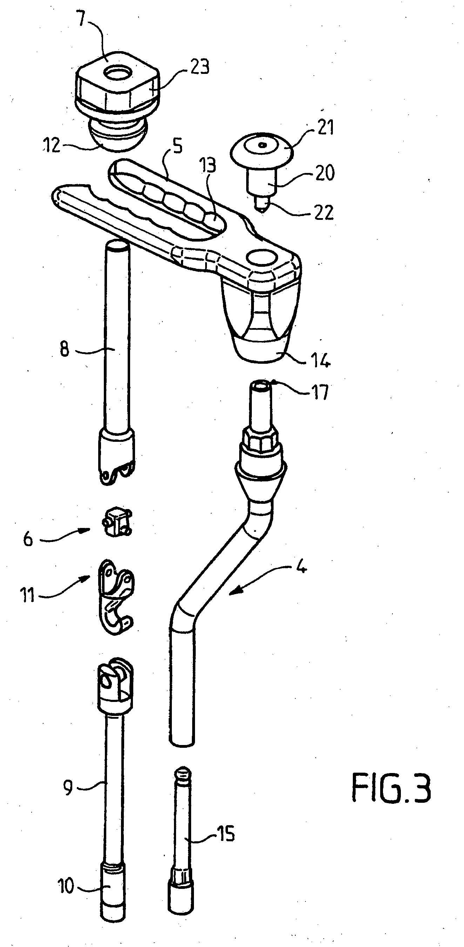

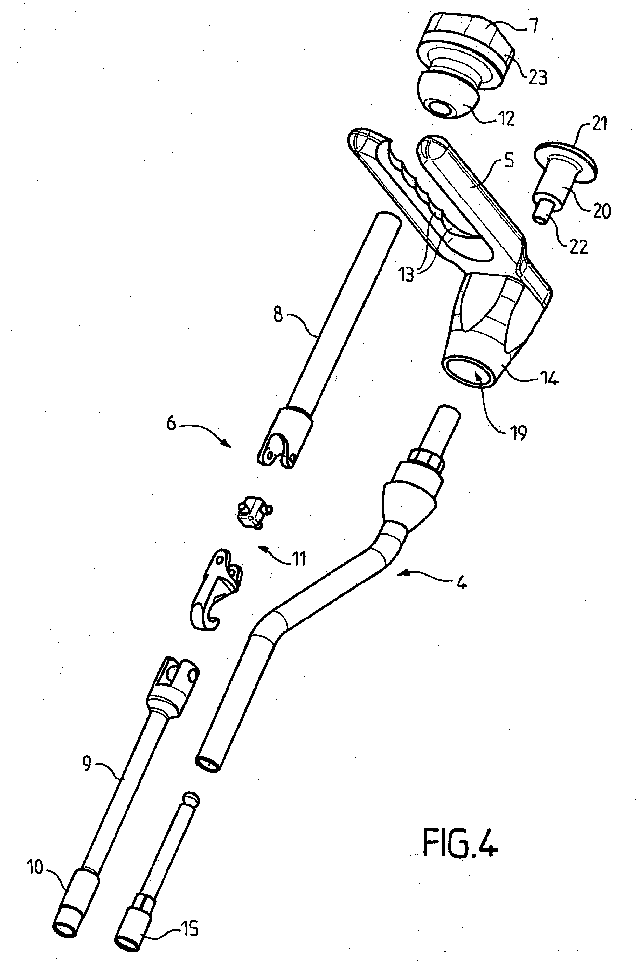

Dans le mode de réalisation représenté sur les figures 2 à 4, le second bras 6 est composé de deux arbres 8, 9 reliés par un élément de connexion 11 possédant au moins un degré de liberté.

Avantageusement, l'élément de connexion 11 possède au moins deux degrés de liberté.

Dans le mode de réalisation représenté sur les figures 2, 3 et 4, l'élément de connexion 11 entre les deux arbres est un cardan.

Notons cependant que l'élément de connexion utilisé peut également être une rotule ou un axe de pivotement.

Dans le mode de réalisation représenté, le moyen d'actionnement 7 du second bras se compose d'un écrou sphérique 12 associé à la traverse 5 et d'un pommeau 23 solidaire de l'écrou sphérique 12.

Notons que le terme sphérique désigne dans ce cas un objet dont une portion s'inscrit dans une sphère.

Le second bras 6 est fileté et le pommeau 23 permet d'entraîner en rotation l'écrou 12 autour du second bras 6. La rotation de l'écrou 12 autour du second bras 6 entraîne la translation du second bras 6 permettant ainsi le déplacement de la seconde vertèbre 1.

La traverse 5 présente une forme de fourchette à deux branches. Au moins un logement sphérique 13 permettant la réception de l'écrou 12 est aménagé entre les deux branches. Les logements sphériques 13 sont agencés pour permettre la rotation de l'écrou 12 dans le logement.

Avantageusement, la traverse 5 possède plusieurs logements sphériques 13 permettant la réception de l'écrou 12 afin de permettre l'ajustement longitudinal de la position du moyen d'actionnement 7. Ainsi, en début d'opération, l'écrou sphérique 12 est disposé dans un des logements selon la position d'utilisation choisie.

Avantageusement, le premier bras 4 est un bras coudé et contre-coudé. La forme particulière de ce bras 4 permet d'éviter les problèmes d'interférence des bras qui découlent de l'anatomie du patient. Par exemple les vertèbres peuvent avoir un angle de lordose qui impose aux vis pédiculaires et donc aux bras 4, 6 une convergence.

Dans le mode de réalisation représenté sur les figures 2 à 4, la traverse 5 est mobile en rotation autour du premier bras 4. La traverse 5 possède un logement 19 permettant de recevoir l'extrémité supérieure du premier bras 4 mobile en rotation dans ledit logement 19. Un bouton poussoir 20 présente une zone de poussée 21 et un corps s'engageant dans le logement 19 de la traverse 5. L'extrémité inférieure 22 du bouton poussoir 20 est de forme sensiblement complémentaire à un logement 17 du premier bras. Ainsi, lorsque l'utilisateur appuie sur le bouton poussoir 20, la rotation de la traverse 5 autour du premier bras 4 est bloquée. Cette mobilité et la possibilité de blocage permettent d'aménager un espace de travail pour la réalisation de travaux sur les vertèbres. Ainsi, il est notamment possible d'insérer un greffon ou une cage intersomatique entre les vertèbres 1, 2.

## Revendications

1. Instrument pour le déplacement d'une vertèbre (1) par rapport à une autre (2) comprenant:
- une potence (3) composée d'une traverse sensiblement horizontale (5) et d'un premier bras (4) dont l'extrémité inférieure (15) possède des moyens d'ancrage (24) à une première vertèbre (2) ; et
- un second bras (6) dont l'extrémité inférieure (10) possède des moyens d'ancrage (25) à une seconde vertèbre (1) et l'extrémité supérieure possède un moyen d'actionnement (7) en translation dudit second bras (6) selon une direction sensiblement perpendiculaire à la traverse (5), ledit moyen d'actionnement étant monté sur la traverse (5), le second bras (6) étant déformable selon au moins un degré de liberté entre l'extrémité inférieure (10) du second bras (6) et son moyen d'actionnement (7), le moyen d'actionnement (7) du second bras (6) se composant d'un écrou (12) associé à la traverse (5), la rotation de l'écrou autour du second bras pourvu d'un filetage entraînant la translation du second bras (6),
**caractérisé en ce que** l'écrou (12) est sphérique et **en ce que** la traverse (5) présente une forme de fourchette à deux branches, au moins un logement sphérique (13) permettant la réception de l'écrou sphérique (12) étant agencé entre ces deux branches, ledit écrou sphérique (12) étant mobile en rotation dans ce logement sphérique (13).

2. Instrument pour le déplacement d'une vertèbre (1) selon la revendication 1, **caractérisé en ce que** le second bras (6) est composé de deux arbres (8, 9) reliés par un élément de connexion (11) possédant au moins un degré de liberté.

3. Instrument pour le déplacement d'une vertèbre (1) selon la revendication 2, **caractérisé en ce que** l'élément de connexion (11) est un cardan.

4. Instrument pour le déplacement d'une vertèbre (1) selon la revendication 2, **caractérisé en ce que** l'élément de connexion (11) est une rotule.

5. Instrument pour le déplacement d'une vertèbre (1) selon la revendication 2, **caractérisé en ce que** l'élément de connexion (11) est un axe de pivotement.

6. Instrument pour le déplacement d'une vertèbre (1) selon la revendication 1, **caractérisé en ce qu'**au moins une partie du second bras (6) est réalisée dans un matériau souple.

7. Instrument pour le déplacement d'une vertèbre (1) selon la revendication 6, **caractérisé en ce qu'**au moins une partie du second bras (6) est un câble.

8. Instrument pour le déplacement d'une vertèbre (1) selon la revendication 6, **caractérisé en ce qu'**au moins une partie du second bras (6) est réalisée dans un matériau polymère.

9. Instrument pour le déplacement d'une (1) vertèbre selon la revendication 6, **caractérisé en ce qu'**au moins une partie du second bras (6) est une pièce métallique déformable.

10. Instrument pour le déplacement d'une vertèbre (1) selon l'une quelconque des revendications 1 à 9, **caractérisé en ce que** le moyen d'actionnement (7) comprend en outre un pommeau (23) solidaire de l'écrou sphérique (12) et permettant d'entraîner ledit écrou (12) en rotation autour du second bras (6).

11. Instrument pour le déplacement d'une vertèbre (1) selon la revendication 10, **caractérisé en ce que** la traverse (5) possède plusieurs logements sphériques (13) permettant la réception de l'écrou (12) afin de permettre l'ajustement longitudinal de la position du moyen d'actionnement (7).

12. Instrument pour le déplacement d'une vertèbre (1) selon l'une quelconque des revendications 1 à 11, **caractérisé en ce que** la potence (3) possède une poignée (14).

13. Instrument pour le déplacement d'une vertèbre (1) selon l'une quelconque des revendications 1 à 12, **caractérisé en ce que** le premier bras (4) est un bras coudé et contre-coudé.

14. Instrument pour le déplacement d'une vertèbre (1) selon l'une quelconque des revendications 1 à 13, **caractérisé en ce que** la traverse (5) est mobile en rotation autour du premier bras (4).

15. Instrument pour le déplacement d'une vertèbre (1) selon la revendication 14, **caractérisé en ce que** la traverse (5) possède un logement (19) agencé pour recevoir un bouton-poussoir (20), ledit bouton-poussoir (20) permettant de bloquer la rotation de la traverse (5) autour du premier bras (4).

16. Instrument pour le déplacement d'une vertèbre (1) selon l'une quelconque des revendications précédentes, **caractérisé en ce que** les moyens d'ancrage (24, 25) des bras (4, 6) sur les vertèbres (1, 2) sont des vis pédiculaires.

17. Instrument pour le déplacement d'une vertèbre (1) selon la revendication 16, **caractérisé en ce que** les vis pédiculaires sont situées dans des logements disposés aux extrémités inférieures (10, 15) des bras.

18. Instrument pour le déplacement d'une vertèbre (1) selon la revendication 16, **caractérisé en ce que** les vis pédiculaires sont positionnées dans des logements de connecteurs (27, 26) solidarisables aux extrémités inférieures (10, 15) des bras.

19. Instrument pour le déplacement d'une vertèbre (1) selon l'une des revendications 16 à 18, **caractérisé en ce que** les vis pédiculaires peuvent également être utilisées pour la fixation d'un élément de liaison longitudinale (28).

## Patentansprüche

1. Instrument zur Bewegung eines Wirbels (1) im Verhältnis zu einem anderen (2), das Folgendes umfasst:
- einen Ausleger (3) bestehend aus einer Querstrebe, die vornehmlich waagerecht ist (5) und einem ersten Arm (4), dessen untere Extremität (15) über Mittel zur Verankerung (24) an einem ersten Wirbel (2) verfügt; und
- einen zweiten Arm (6), dessen untere Extremität (10) über Mittel zur Verankerung (25) an einem zweiten Wirbel (1) verfügt und die obere Extremität verfügt über ein Auslösemittel (7) zur Bewegung des genannten zweiten Arms (6), entsprechend einer vornehmlich senkrechten Richtung zu dem Querbalken (5), wobei das genannte Auslösemittel auf dem Querbalken (5) montiert ist, der zweit Arm (6) verformbar entsprechend mindestens einem Grad zwischen der unteren Extremität (10) und dem zweiten Arm (6) ist und sein Auslösemittel (7), das Auslösemittel des zweiten Arms (6) aus einer Mutter (12) besteht, die mit dem Querbalken (5) verbunden ist, die Drehung der Mutter um den zweiten Arm mit Gewinde die Verschiebung des zweiten Arms (6) auslöst,
**dadurch gekennzeichnet, dass** die Mutter (12) sphärisch ist und dass der Querbalken (5) die Form einer zweizinkigen Gabel aufweist mit mindestens einer sphärischen Aufnahme (13), die die Aufnahme der sphärischen Mutter (12) ermöglicht, die in dieser sphärischen Aufnahme (13) frei beweglich ist.

2. Instrument zur Bewegung eines Wirbels (1) gemäß dem Anspruch 1, **dadurch gekennzeichnet, dass** der zweite Arm (6) aus zwei Verzweigungen (8,9) besteht, die durch ein Verbindungselement (11) mit mindestens einem Grad Freiheit verbunden sind.

3. Instrument zur Bewegung eines Wirbels (1) gemäß dem Anspruch 1, **dadurch gekennzeichnet, dass** das Verbindungselement (11) eine Kardanwelle ist.

4. Instrument zur Bewegung eines Wirbels (1) gemäß dem Anspruch 1, **dadurch gekennzeichnet, dass** das Verbindungselement (11) ein Kugelgelenk ist.

5. Instrument zur Bewegung eines Wirbels (1) gemäß dem Anspruch 1, **dadurch gekennzeichnet, dass** das Verbindungselement (11) eine Schwenkachse ist.

6. Instrument zur Bewegung eines Wirbels (1) gemäß dem Anspruch 1, **dadurch gekennzeichnet, dass** mindestens ein Teil des zweiten Arms (6) aus biegsamem Material gefertigt ist.

7. Instrument zur Bewegung eines Wirbels (1) gemäß dem Anspruch 1, **dadurch gekennzeichnet, dass** mindestens ein Teil des zweiten Arms (6) ein Kabel ist.

8. Instrument zur Bewegung eines Wirbels (1) gemäß dem Anspruch 1, **dadurch gekennzeichnet, dass** mindestens ein Teil des zweiten Arms (6) aus einem Polymermaterial besteht.

9. Instrument zur Bewegung eines Wirbels (1) gemäß dem Anspruch 1, **dadurch gekennzeichnet, dass** mindestens ein Teil des zweiten Arms (6) aus einem verformbaren Metallstück besteht.

10. Instrument zur Bewegung eines Wirbels (1) gemäß einem der Ansprüche 1 bis 9, **dadurch gekennzeichnet, dass** das Auslösemittel (7) des Weiteren einen mit der sphärischen Mutter solidarischen Knauf (23) umfasst und es ermöglicht, die genannte Mutter (12) um den zweiten Arm (6) zu drehen.

11. Instrument zur Bewegung eines Wirbels (1) gemäß dem Anspruch 10, **dadurch gekennzeichnet, dass** der Querbalken (5) mehrere sphärische Aufnahmen (13) umfasst, die die Aufnahme der Mutter (12) ermöglichen, um die Längsanpassung der Position des Auslösemittels (7) zu ermöglichen.

12. Instrument zur Bewegung eines Wirbels (1) gemäß einem der Ansprüche 1 bis 11, **dadurch gekennzeichnet, dass** der Ausleger (3) einen Knauf (14) umfasst.

13. Instrument zur Bewegung eines Wirbels (1) gemäß einem der Ansprüche 1 bis 12, **dadurch gekennzeichnet, dass** der erste Arm (4) gekrümmt und gegengekrümmt ist.

14. Instrument zur Bewegung eines Wirbels (1) gemäß einem der Ansprüche 1 bis 13, **dadurch gekennzeichnet, dass** der Querbalken (5) beweglich um den ersten Arm (4) drehbar ist.

15. Instrument zur Bewegung eines Wirbels (1) gemäß dem Anspruch 14, **dadurch gekennzeichnet, dass** der Querbalken (5) eine Aufnahme (19) aufweist, die eine Drucktaste (20) aufnimmt, wobei die besagte Drucktaste (20) es ermöglicht, die Rotation des Querbalkens (5) um den ersten Arm (4) zu blockieren.

16. Instrument zur Bewegung eines Wirbels (1) gemäß einem der vorangegangenen Ansprüche, **dadurch gekennzeichnet, dass** die Mittel zur Verankerung (24,25) der Arme (4, 6) auf den Wirbeln (1, 2) Pedikelschrauben sind.

17. Instrument zur Bewegung eines Wirbels (1) gemäß dem Anspruch 16, **dadurch gekennzeichnet, dass** die Pedikelschrauben sich in Aufnahmen an den unteren Extremitäten (10, 15) der Arme befinden.

18. Instrument zur Bewegung eines Wirbels (1) gemäß dem Anspruch 16, **dadurch gekennzeichnet, dass** die Pedikelschrauben sich in Aufnahmen der Verbindungen (27, 26) befinden, die solidarisch zu den unteren Extremitäten (10, 15) der Arme gemacht werden können.

19. Instrument zur Bewegung eines Wirbels (1) gemäß einem der Ansprüche 16 bis 18, **dadurch gekennzeichnet, dass** die Pedikelschrauben auch für die Fixierung eines Längsverbindungselements verwendet werden können.

## Claims

1. An instrument for moving one vertebra (1) with respect to another (2), comprising:
- a bracket (3) composed of a substantially horizontal cross member (5) and a first arm (4), the bottom end (15) of which has means (24) for anchoring to a first vertebra (2); and
- a second arm (6), the bottom end (10) of which has means (25) for anchoring to a second vertebra (1) and the top end of which has a means (7) for translational actuation of said second arm (6) in a direction substantially perpendicular to the cross member (5), said actuation means being mounted on the cross member (5), the second arm (6) being deformable in accordance with at least one degree of freedom between the bottom end (10) of the second arm (6) and its actuation means (7), the means (7) for actuating the second arm (6) being composed of a nut (12) associated with the cross member (5), the rotation of the nut about the second arm provided with a thread causing the translation of the second arm (6),
**characterised in that** the nut (12) is spherical and **in that** the cross member (5) is in the form of a fork with two arms, at least one spherical housing (13) for receiving the spherical nut (12) being arranged between these two arms, said spherical nut (12) being able to rotate in this spherical housing (13).

2. An instrument for moving a vertebra (1) according to claim 1, **characterised in that** the second arm (6) is composed of two shafts (8, 9) connected by a connection element (11) having at least one degree of freedom.

3. An instrument for moving a vertebra (1) according to claim 2, **characterised in that** the connection element (11) is a universal joint.

4. An instrument for moving a vertebra (1) according to claim 2, **characterised in that** the connection element (11) is a swivel.

5. An instrument for moving a vertebra (1) according to claim 2, **characterised in that** the connection element (11) is a pivot spindle.

6. An instrument for moving a vertebra (1) according to claim 1, **characterised in that** at least part of the second arm (6) is produced from a flexible material.

7. An instrument for moving a vertebra (1) according to claim 6, **characterised in that** at least part of the second arm (6) is a cable.

8. An instrument for moving a vertebra (1) according to claim 6, **characterised in that** at least part of the second arm (6) is produced from a polymer material.

9. An instrument for moving a vertebra (1) according to claim 6, **characterised in that** at least part of the second arm (6) is a deformable metal part.

10. An instrument for moving a vertebra (1) according to any one of claims 1 to 9, **characterised in that** the actuation means (7) further comprises a knob (23) secured to the spherical nut (12) and making it possible to rotate said nut (12) about the second arm (6).

11. An instrument for moving a vertebra (1) according to claim 10, **characterised in that** the cross member (5) has several spherical housings (13) for receiving the nut (12) in order to allow longitudinal adjustment of the position of the actuation means (7).

12. An instrument for moving a vertebra (1) according to any one of claims 1 to 11, **characterised in that** the bracket (3) has a handle (14).

13. An instrument for moving a vertebra (1) according to any one of claims 1 to 12, **characterised in that** the first arm (4) is an angled and cranked arm.

14. An instrument for moving a vertebra (1) according to any one of claims 1 to 13, **characterised in that** the cross member (5) is able to rotate about the first arm (4).

15. An instrument for moving a vertebra (1) according to claim 14, **characterised in that** the cross member (5) has a housing (19) arranged so as to receive a push button (20), said push button (20) making it possible to lock the rotation of the cross member (5) about the first arm (4).

16. An instrument for moving a vertebra (1) according to any one of the preceding claims, **characterised in that** the means (24, 25) for anchoring the arms (4, 6) on the vertebrae (1, 2) are pedicular screws.

17. An instrument for moving a vertebra (1) according to claim 16, **characterised in that** the pedicular screws are situated in housings disposed at the bottom ends (10, 15) of the arms.

18. An instrument for moving a vertebra (1) according to claim 16, **characterised in that** the pedicular screws are positioned in housings of connectors (27, 26) able to be secured to the bottom ends (10, 15) of the arms.

19. An instrument for moving a vertebra (1) according to one of claims 16 to 18, **characterised in that** the pedicular screws can also be used for fastening a longitudinal connection element (28).
